(19) Europäisches Patentamt
European Patent Office
Office européen des brevets

(11) Veröffentlichungsnummer: **0 308 785 B1**

(12) # EUROPÄISCHE PATENTSCHRIFT

(45) Veröffentlichungstag der Patentschrift: **13.05.92**

(51) Int. Cl.[5]: **C07D 211/86**, C07D 211/90, C07D 401/12, A61K 31/445

(21) Anmeldenummer: **88114979.3**

(22) Anmeldetag: **14.09.88**

(54) **Dihydropyridinether, Verfahren zu ihrer Herstellung und ihre Verwendung.**

(30) Priorität: **25.09.87 DE 3732380**

(43) Veröffentlichungstag der Anmeldung:
**29.03.89 Patentblatt 89/13**

(45) Bekanntmachung des Hinweises auf die Patenterteilung:
**13.05.92 Patentblatt 92/20**

(84) Benannte Vertragsstaaten:
**AT BE CH DE ES FR GB GR IT LI NL SE**

(56) Entgegenhaltungen:
**DE-A- 2 210 650**
**DE-A- 3 420 784**

(73) Patentinhaber: **BAYER AG**
**W-5090 Leverkusen 1 Bayerwerk(DE)**

(72) Erfinder: **Franckowiak, Gerhard, Dr.**
**Henselweg 10**
**W-5600 Wuppertal 1(DE)**
Erfinder: **Bechem, Martin, Dr.**
**Obere Bergerheide 4**
**W-5600 Wuppertal 1(DE)**
Erfinder: **Gross, Rainer, Prof. Dr.**
**Platzhofstrasse 23**
**W- 5600 Wuppertal 1(DE)**
Erfinder: **Hebisch, Siegfried, Dr.**
**Herzogstrasse 129**
**W-4200 Oberhausen 11(DE)**
Erfinder: **Schramm, Matthias, Dr.**
**Paffrather Strasse 38**
**W-5000 Köln 80(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.08/2.19/2.0)

## Beschreibung

Die Erfindung betrifft neue Dihydropyridinether, mehrere Verfahran zu ihrer Herstellung und ihre Verwendung in Arzneimitteln, insbesondere in kreislaufbeeinflussenden Arzneimitteln.

Aus DEA-3 420 784 und DEA-2 210 650 sind bereits Dihydropyridine mit kreislaufbeeinflussender Wirkung bekannt, die in 3- und 5-Position jeweils Carboxy, Carbonyl, Nitro oder Cyano Substituente tragen. Die erfindungsgemäßen Aryloxy- bzw. Alkoxyderivate von Dihydropyridinen sind durch ihre Ethergruppe strukturell nicht mit den bekannten Verbindungen vergleichbar.

Die vorliegende Erfindung betrifft neue Dihydropyridinether der allgemeinen Formel (I)

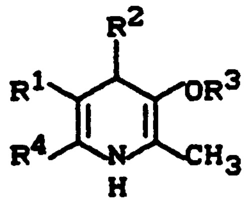

(I),

in welcher

$R^1$ - für Cyano, Nitro oder
- für eine Gruppe $-COOR^5$ steht,
worin

$R^5$ - geradkettiges, verzweigtes oder cyclisches Alkyl mit bis zu 6 Kohlenstoffatomen bedeutet, das durch ein Sauerstoff in der Kette unterbrochen sein kann, und das ein- oder mehrfach gleich oder verschieden durch Halogen, Cyano, Hydroxy, Phenyl, Phenoxy oder durch eine Gruppe der Formel

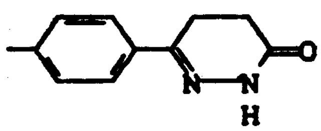

substituiert sein kann,

$R^2$ - für Aryl mit 6 bis 12 Kohlenstoffatomen steht, das bis zu 3-fach gleich oder verschieden durch Halogen, Cyano, Nitro, Trifluormethyl, Trifluormethoxy, Trifluormethylthio, Alkyl mit bis zu 4 Kohlenstoffatomen oder Alkoxy mit bis zu 4 Kohlenstoffatomen substituiert sein kann,

$R^3$ - für Aryl mit 6 bis 12 Kohlenstoffatomen steht, oder
- für geradkettiges, verzweigtes oder cyclisches Alkyl mit bis zu 6 Kohlenstoffatomen steht
und

$R^4$ - für geradkettiges Alkyl mit bis 2 bis 4 Kohlenstoffatomen oder
- für Amino steht

und deren physiologisch unbedenkliche Salze.

Physiologisch unbedenkliche Salze können Salze der erfindungsgemäßen Verbindungen mit anorganischen oder organischne Säuren sein. Als Beispiele seien genannt: Hydrohalogenide wie zum Beispiel Hydrochloride oder Hydrobromide, Hydrogensulfate, Sulfate, Hydrogenphosphate, Phosphate, oder Acetate, Maleate, Fumarate, Citrate, Tartrate, Lactate oder Benzoate.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Die erfindungsgemäßen Verbindungen sind neu und besitzen wertvolle pharmakologische Eigenschaften. Sie beeinflussen den Blutdruck und können somit zur Bekämpfung von Kreislauferkrankungen eingesetzt werden.

Bevorzugte Verbindungen der allgemeinen Formel (I) sind solche
in denen

$R^1$ - für Nitro oder
- für eine Gruppe $-COOR^5$ steht,
worin

$R^5$ - geradkettiges oder verzweigtes Alkyl mit bis zu 4 Kohlenstoffatomen bedeutet, das durch ein Sauerstoffatom in der Kette unterbrochen sein kann, und das durch Fluor, Chlor, Brom, Cyano, Hydroxy, Phenyl oder durch eine Gruppe der Formel

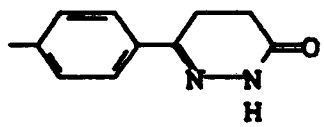

substituiert sein kann,

$R^2$ - für Phenyl steht, das bis zu 2-fach gleich oder verschieden durch Fluor, Chlor, Brom, Nitro, Trifluormethyl, Trifluormethoxy, Methyl oder Methoxy substituiert sein kann

$R^3$ - für Phenyl steht, oder
- für geradkettiges oder verzweigtes Alkyl mit bis zu 4 Kohlenstoffatomen,
und

$R^4$ - für geradkettiges Alkyl mit bis zu 4 Kohlenstoffatomen oder
- für Amino steht,

und deren physiologisch unbedenkliche Salze.

Besonders bevorzugte Verbindungen der allgemeinen Formel (I) sind solche,
in denen

$R^1$ - für Nitro oder
- für eine Gruppe $-COOR^5$ steht
worin

$R^5$ - für geradkettiges oder verzweigtes Alkyl mit bis zu 3 Kohlenstoffatomen steht, das durch eine Gruppe der Formel

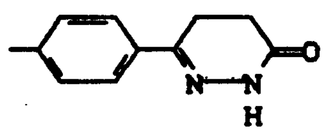

substituiert sein kann,

$R^2$ - für Phenyl steht, das bis zu 2-fach gleich oder verschieden durch Chlor, Nitro oder Trifluormethyl substituiert sein kann,

$R^3$ - für Phenyl oder Methyl,
und

$R^4$ - für Methyl oder
- für Amino steht

und deren physiologisch unbedenkliche Salze.

Die erfindungsgemäßen Verbindungen der allgemeinen Formel (I),
in welcher
$R^1$, $R^2$, $R^3$ und $R^4$ die oben angegebene Bedeutung haben,
erhält man,
indem man
[A] Aldehyde der allgemeinen Formel (II)

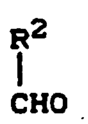

(II)

in welcher
$R^2$ - die oben angegebene Bedeutung hat
und Ketone der allgemeinen Formel (III)

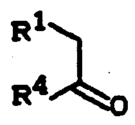

(III)

in welcher
$R^1$ und $R^4$ die oben angegebene Bedeutung haben,
oder deren Knoevenagel-Kondensationsprodukte (Ylidenverbindungen) der allgemeinen Formel (IV)

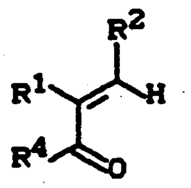

(IV)

in welcher
$R^1$, $R^2$ und $R^4$ die oben angegebene Bedeutung haben,
mit Enaminen der allgemeinen Formel (V)

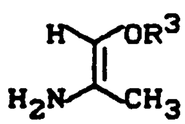

(V)

in welcher
$R^3$ die oben angegebene Bedeutung hat,
in inerten Lösemitteln umsetzt,
oder indem man
[B] Aldehyde der allgemeinen Formel (II) und Ketone der allgemeinen Formel (VI)

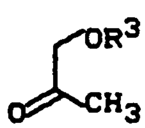

(VI)

in welcher
$R^3$ die oben angegebene Bedeutung hat,
oder deren Ylidenverbindungen der allgemeinen Formel (VII)

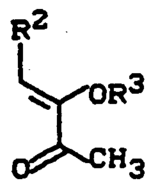

(VII)

in welcher
$R^2$ und $R^3$ die oben angegebene Bedeutung haben,
mit Ammoniak und Ketonen der allgemeinen Formel (III), oder mit Enaminen der allgemeinen Formel (VIII)

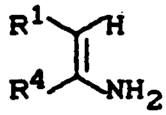

(VIII)

in welcher
$R^1$ und $R^4$ die oben angegebene Bedeutung haben,
in inerten Lösemitteln umsetzt.

Je nach Art der verwendeten Ausgangsstoffe können die Synthesevarianten A bzw. B für die erfindungsgemäßen Verbindungen durch folgende Formelschemata wiedergegeben werden:

[A]

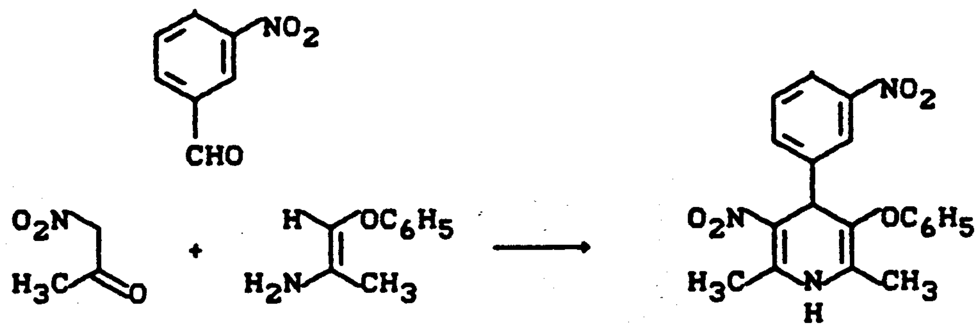

bzw.

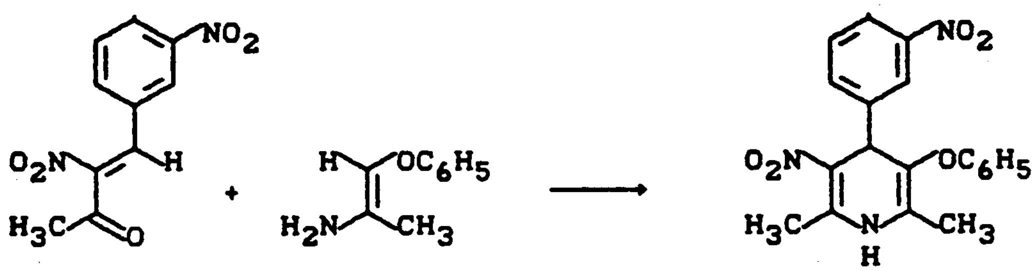

[B]

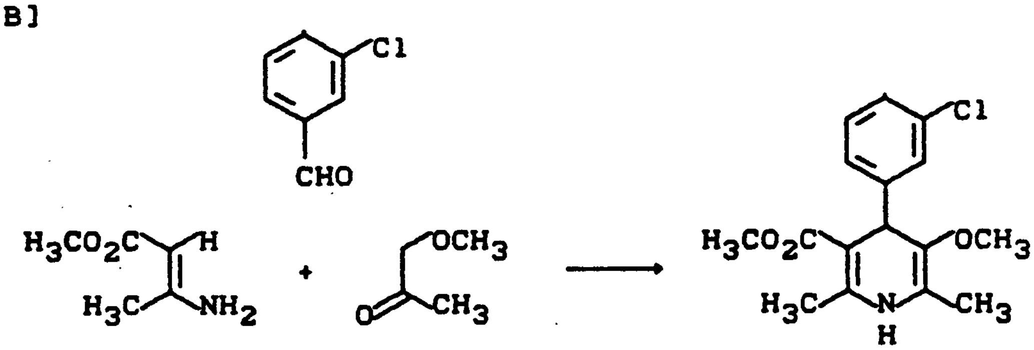

bzw.

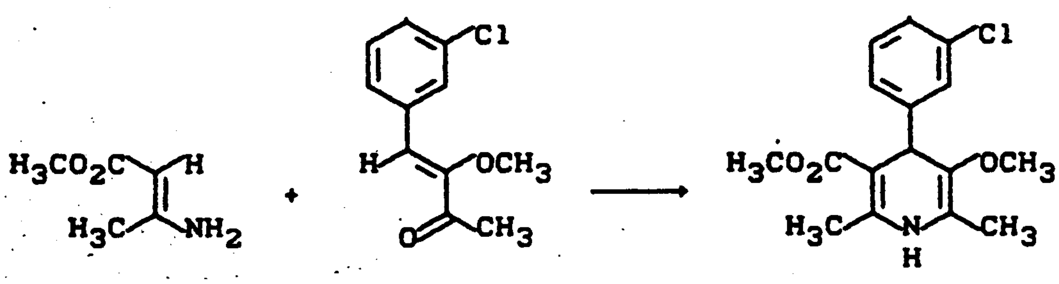

Als Lösemittel kommen alle inerten organischen Lösemittel in Frage. Hierzu gehören vorzugsweise Alkohole wie Methanol, Ethanol, Propanol oder Isopropanol, oder Ether wie Diethylether, Dioxan, Tetrahydrofuran, Glykolmonomethylether oder Glykoldimethylether, oder Amide wie Dimethylformamid oder Hexamethylphosphorsäuretriamid, oder Eisessig, Acetonitril, Dimethylsulfoxid oder Pyridin, Ebenso ist es möglich, Gemische der genannten Lösemittel einzusetzen.

Die Reaktionstemperaturen können in einem größeren Bereich variiert werden. Im allgemeinen arbeitet man zwischen +20 °C und +150 °C, vorzugsweise zwischen +20 °C und +100 °C, insbesondere der Siedetemperatur des jeweiligen Lösemittels.

Die Umsetzung kann bei Normaldruck, aber auch bei erhöhtem oder erniedrigtem Druck durchgeführt werden. Im allgemeinen arbeitet man unter Normaldruck.

Bei der Durchführung der erfindungsgemäßen Verfahren A und B ist das Verhältnis der an der Reaktion beteiligten Stoffe beliebig. Im allgemeinen arbeitet man mit molaren Mengen der Reaktanden. Es hat sich aber als zweckmäßig erwiesen, Nitroaceton bzw. das Nitroaceton-Ammoniak-Additionsprodukt im bis zu 20-fachen, bevorzugt bis zu 10-fachen Überschuß einzusetzen.

Die als Ausgangsstoffe eingesetzten Aldehyde der allgemeinen Formel (II) sind bekannt oder können nach bekannten Methoden hergestellt werden [E. Mosettig, Organic Reactions Vol. III, 218 (1954)].

Die als Ausgangsstoffe eingesetzten Ketone der allgemeinen Formel (III) sind bekannt oder können nach bekannten Methoden hergestellt werden [D. Borrmann, Houben-Weyls "Methoden der organischen Chemie" Bd. VII/4, 230 (1968); N. Levy, C.W. Scaife, J. Chem. Soc. (London) 1946, 1100; C.D. Hurd, M.E. Nilson, J. Org. Chem. 20, 927 (1955)].

Die als Ausgangsstoffe eingesetzten Ketone der allgemeinen Formel (VI) sind bekannt oder können nach bekannten Methoden hergestellt werden [Tegner, Acta Chem. Scand. 6, 782 (1952); Reppe et al., Liebigs Ann. Chem. 596, 1 (1955); D. Gauthier, Annales de Chimie [8] 16, 318; Stroemer et al., Chem. Ber. 28, 1253 (1895); St. Welin, Chem. Ber. 35, 3553 (1902)].

Die als Ausgangsstoffe eingesetzten Enamine der allgemeinen Formeln (V) und (VIII) sind bekannt oder können nach bekannten Methoden hergestellt werden [F.A. Glickman, A.C. Cope, J. Am. Chem. Soc. 67, 1017 (1945); H. Boehme, K.-H. Weisel, Arch. Pharm. 310, 30 (1977)].

Die als Ausgangsstoffe eingesetzten Knoevenagel-Kondensationsprodukte der Formel (IV) (Ylidenverbindungen) sind teilweise bekannt oder können nach an sich bekannten Methoden hergestellt werden [G. Jones, "The Knoevenagel Condensation" Organic Reactions XV, 204 (1976); A. Dornow, W. Sassenberg, Liebigs Ann. Chem., 602, 14 (1957)].

Die als Ausgangsstoffe eingesetzten Ylidenverbindungen der allgemeinen Formel (VII) sind teilweise bekannt oder können nach bekannten Methoden hergestellt werden [W. Grell, H. Machleit, Liebigs Ann. Chem. 699, 53 (1966); V. Rosnati, A. Salimbeni, Gazz. Chim. Ital. 107, 271 (1977)].

Die vorstehenden Herstellungsverfahren sind lediglich zur Verdeutlichung angegeben. Die Herstellung der Verbindungen der Formel I ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen beeinflussen die Kontraktionskraft des Herzens und den Tonus der glatten Muskulatur. Sie können deshalb in Arzneimitteln zur Beeinflussung des pathologisch veränderten Blutdrucks, als Koronartherapeutika und zur Behandlung der Herzinsuffizienz, eingesetzt werden. Darüberhinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herzwirkung wurde an isolierten Vorhöfen von Meerschweinschenherzen gefunden.

Dazu werden die linken Vorhöfe von Meerschweinchenherzen isoliert, und in ein auf 32 °C thermostatisiertes Organbad gehängt. Als Inkubationsmedium dient Krebs-Henseleit-Lösung mit folgender Zusammensetzung (118,5 mmol/l NaCl, 4,75 mmol/l KCl, 1,19 mmol/l $KH_2PO_4$, 119 mmol/l $MgSO_4$, 25 mmol/l $NaHCO_3$, 0,013 mmol/l NaEDTA, 1,8 mmol/l $CaCl_2$) unter Zusatz von 10 mmol/l Glukose als energielieferndes Substrat. Die Lösung wird mit Carbogen (95 % $CO_2$, 5 % $O_2$) zur Aufrechterhaltung eines pH-Wertes von 7,4 begast. Die linken Vorhöfe werden unter Einstellung eines bestimmten Grundtones in das Organbad eingespannt, und die Spannung mittels eines Kraftaufnehmers registriert. Unter periodischer elektrischer Reizung werden die dabei erfolgenden Kontraktionen fortlaufend auf einem Schnellschreiber aufgezeichnet. In Gegenwart der jeweiligen erfindungsgemäßen Verbindungen kommt es dabei zu einer prozentualen Veränderung gegenüber dem gleich 100 % gesetzten Ausgangswert:

| Beispiel-Nr. | Konzentration | prozentuale Änderung der Kontraktionskraft |
|---|---|---|
| 5 | $10^{-2}$ | + 23 |
| 7 | $10^{-2}$ | + 30 |

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z. B. Ethylalkohol, Glycerin), Trägerstoffe, wie z.B. natürliche Gesteinsmehle (z.B. Kaoline, Tonerden, Talkum, Kreide), synthetische

Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

## Herstellungsbeispiele

### Beispiel 1

1-Diethylphosphono-1-methoxyaceton

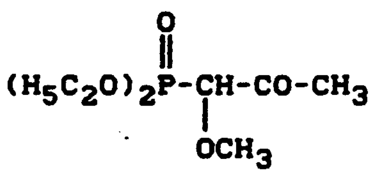

In 2 mol 1,1-Dimethoxyaceton werden bei max. 60 °C 2 mol Acetylbromid zugetropft und 1 h bei 60 °C nachgerührt. Das 1-Brom-1-methoxy-aceton wird im Vakuum destilliert (55% / 11 Torr); man erhält 271 g , die tropfenweise mit 1,614 mol Triethylphosphit versetzt werden. Danach wird 1 h bei 120 °C und 1 h bei 140 °C Ethylbromid abdestilliert. Der Rückstand wird im Ölpumpenvakuum destilliert (88 °C / 0,05 Torr) (Ann. Chem. 696, 59 (1966))

Ausbeute: 280,4 g (62,5% der Theorie)

Beispiel 2

2-Methoxy-1-(2-trifluormethylphenyl)but-1-en-3-on

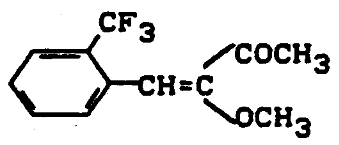

Zu einer Suspension von 0,106 mol Natriumhydrid in 200 ml absolutem Dioxan tropft man in 0,5 h 0,102 mol Phosphonat aus Beispiel 1, läßt 0,5 h nachrühren und tropft dann unter Kühlung bei Raumtemperatur 0,100 Mol o-Trifluormethylbenzaldehyd zu. Man läßt 3 h nachrühren, gibt 2 ml Wasser in 20 ml Dioxan zu, dampft das Lösemittel im Vakuum ab und fraktioniert den Rückstand im Ölpumpenvakuum.
Sdp.: 60 - 65 ° C (0,01 Torr)
Ausbeute: 12,2 g (50% der Theorie)
Das Produkt liegt laut NMR als cis/trans-Gemisch im Verhältnis 2:3 vor.

Beispiel 3

1,4-Dihydro-2,6-dimethyl-3-methoxy-5-nitro-4-(2-trifluormethylphenyl)pyridin

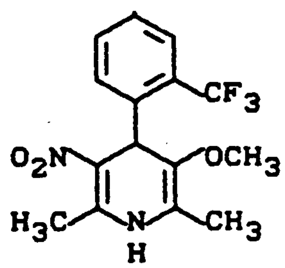

50 mmol Methoxybenzalaceton aus Beispiel 2 werden in 30 ml Isopropanol bei 60 ° C über 4 h in Portionen mit insgesamt 0,15 mol Nitroaceton-Ammoniak-Addukt versetzt. Es wird weitere 4 h bei 60 ° C gerührt. Anschließend wird das Lösemittel abrotiert, der Rückstand wird an Kieselgel mit Chloroform chromatographiert. Die vereinigten Produktfraktionen werden aus Isopropanol/Petrolether 1:1 umkristallisiert.
Ausbeute: 2,6 g (16% der Theorie)
Schmp.: 201 ° C

Beispiel 4

2-Amino-1-phenoxy-propen

0,2 Mol Phenoxyaceton werden in 200 ml Tetrahydrofuran bei Rückflußtemperatur unter Zusatz von 0,2 g p-Toluolsulfonsäure mit Ammoniakgas gesättigt. Man läßt 24 Stunden bei Raumtemperatur stehen und dampft das Lösemittel dann im Vakuum ab. Der Eindampfrückstand wird roh in die Reaktion von Beispiel 5 B eingesetzt.

Beispiel 5

1,4-Dihydro-2,6-dimethyl-5-nitro-3-phenoxy-4-phenylpyridin

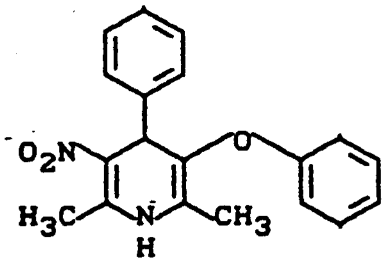

A) 10 mmol 2-Phenoxy-1-phenyl-but-1-en-3-on (hergestellt analog Beispiel 3) werden in 30 ml Isopropanol bei 40 ° C portionsweise mit 30 mmol Nitroaceton-Ammoniak-Additionsverbindung versetzt. Es wird 1 h auf 60 ° C und unter Zusatz von 1 ml wäßrigem Ammoniak 8 h zum Rückfluß erhitzt. Das Lösemittel wird abgedampft und der Rückstand am Kieselgel mit Chloroform chromatographiert.
Ausbeute: 12% der Theorie
Schmp.: Harz
B) 20 mmol 2-Nitro-1-phenyl-but-1-en-3-on werden in 50 ml Isopropanol mit 25 mmol rohem 2-Amino-1-phenoxy-propen versetzt und 8 h bei 60 ° C gerührt. Das Lösemittel wird abgedampft, der Rückstand an Kieselgel mit Chloroform chromatographiert.
Ausbeute: 15% der Theorie
Schmp.: Harz

Beispiel 6

2-Phenoxy-1-(2-trifluormethyl-phenyl)but-1-en-3-on

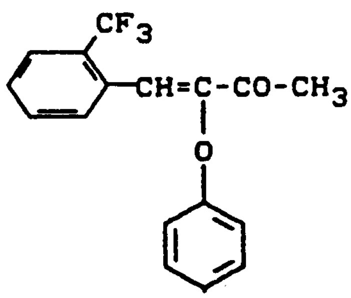

0,2 mol Phenoxyaceton und 0,25 mol o-Trifluormethylbenzaldehyd werden in 8 l entgastem Wasser mit 16 g Natriumhydroxid 3 Tage unter Luftabschluß intensiv gerührt. Der Ansatz wird mit Methylenchlorid angeschüttelt, die organische Phase wird getrocknet, eingeengt, mit Petrolether digeriert und dann aus Ethanol umkristallisiert.
Ausbeute: 45,2% der Theorie
Schmp.: 89 ° C

Beispiel 7

1,4-Dihydro-2,6-dimethyl-5-nitro-3-phenoxy-4-(2-(trifluormethylphenyl)pyridin

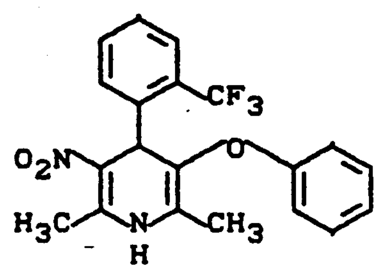

Analog Beispiel 6 wird aus 2-Phenoxy-1-(2-trifluormethylphenyl)-but-1-en-3-on und Nitroaceton-Ammoniak-Additionsverbindung nach Chromatographie an Kieselgel die Titelverbindung erhalten.
Ausbeute: 18% der Theorie
Schmp.: 123 ° C

Beispiel 8

4-Oxo-4-[4-(tetrahydropyran-2-yl-oxymethyl)phenyl]-buttersäure

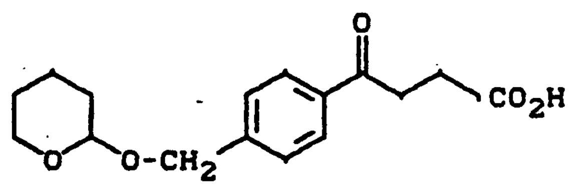

Unter Schutzgas werden 12,75 g (0,53 mol) Magnesium-Späne in 200 ml Tetrahydrofuran mit wenig Iod aktiviert, dann läßt man 135 g p-Brombenzyl-tetrahydropyranylether langsam zutropfen. Die Reaktion verläuft stark exotherm. Zur vollständigen Umsetzung läßt man 2 h unter Erwärmen nachrühren. Die Lösung wird auf -80 ° C gekühlt und 53 g Bernsteinsäureanhydrid, suspendiert in 200 ml Tetrahydrofuran werden portionsweise zugegeben. Es wird 2 h nachgerührt, dann mit 4 l Wasser hydrolysiert und mit 2 N Natronlauge auf pH 8 eingestellt. Es wird mit Methylenchlorid gewaschen. Die wäßrige Phase wird mit Citronensäure auf pH 4,5 eingestellt. Das Produkt wird mit Methylenchlorid extrahiert, getrocknet und durch Zugabe von Petrolether ausgefällt.
Ausbeute: 59 g (40,4% der Theorie)
Schmp.: 97 ° C

Beispiel 9

4-(6-Oxo-1,4,5,6-tetrahydropyridazin-3-yl)benzylalkohol

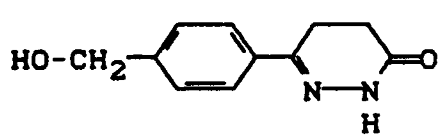

50 g 4-Oxo-4-[4-(tetrahydropyran-2-yl-oxymethyl)-phenyl]-buttersäure werden in 240 ml Wasser suspendiert und mit 10 ml Hydrazinhydrat 3 h bei 90 ° C verrührt. Nach dem Abkühlen saugt man die ausgefallenen Kristalle ab. Diese werden in 500 ml Tetrahydrofuran gelöst und mit je 100 ml Eisessig, 1 N Salzsäure und Wasser versetzt. Man läßt 24 h bei Raumtemperatur stehen, destilliert das Tetrahydrofuran ab und

extrahiert den Rückstand mit Methylenchlorid. Dabei fällt das Produkt bereits teilweise aus und wird abgesaugt. Die Methylenchloridphase wird aufgearbeitet. Beide Kristallisate werden verunreinigt.
Ausbeute: 22,7 g (64% der Theorie)
Schmp.: 188 °C

Beispiel 10

β-Aminocrotonsäure-[4-(6-oxo-1,4,5,6-tetrahydropyridazin-3-yl)benzyl]ester

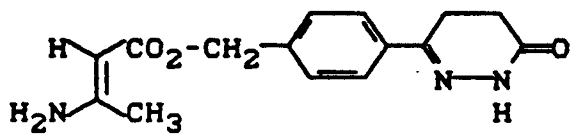

20 g 4-(6-Oxo-1,4,5,6-tetrahydropyridazin-3-yl)benzylalkohol werden in 50 ml Tetrahydrofuran suspendiert und nach Zugabe von 100 mg 4-Dimethylaminopyridin unter Rückfluß mit 10,7 Diketen umgesetzt. Man läßt 2 h nachreagieren, sättigt bei Rückflußtemperatur mit Ammoniakgas, läßt über Nacht erkalten, dampft das Tetrahydrofuran ab und kocht den Rückstand mit Isopropanol aus.
Ausbeute: 17,5 g (92% der Theorie) / Schmp.: 183 °C
Schmp.: 183 °C

Beispiel 11

1,4-Dihydro-2,6-dimethyl-5-phenoxy-4-(2-trifluormethylphenyl)-pyridin-3-carbonsäure-(4-(6-oxo-1,4,5,6-tetrahydropyridazin-3-yl-benzyl)-ester

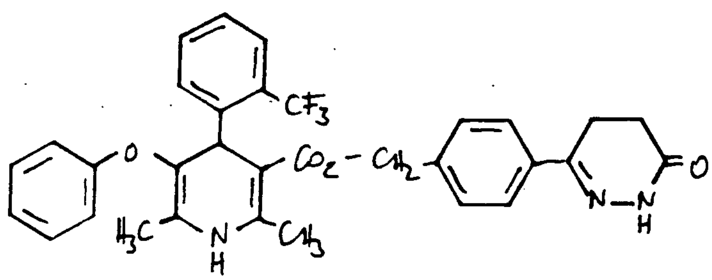

10 mMol 2-Phenoxy-1-(2-trifluormethylphenyl)-but-1-en-3-on und 10 mMol 3-Aminocrotonsäure-[4-(6-oxo-1,4,5,6-tetrahydropyridazin-3-yl)-benzyl]-ester werden in 20 ml Isopropanol 6 Stunden zum Rückfluß erhitzt. Der rohe Eindampfrückstand wird an Kieselgel mit Chloroform plus 6% Methanol chromatographiert.
Schmp.: 168 °C
Ausbeute: 8% der Theorie

Beispiel 12

2-Amino-1,4-dihydro-6-methyl-5-phenoxy-4-phenyl-pyridin-3-carbonsäureethylester

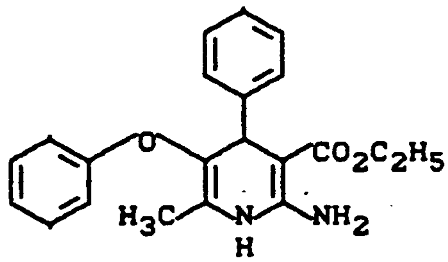

Zu 10 mmol 3-Phenoxy-1-phenyl-but-1-en-3-on werden in 20 ml Ethanol bei Rückflußtemperatur eine Lösung von 25 mmol Amidinoessigester-hydrochlorid und 25 mmol Natriummethylat in 20 ml Ethanol zugetropft. Man erhitzt noch 2 h zum Rückfluß, dampft das Lösemittel ab und chromatographiert das Rohprodukt an Kieselgel mit Chloroform. Das Produkt wird als Harz isoliert.
Ausbeute: 6% der Theorie

**Patentansprüche**

**Patentansprüche für folgende Vertragsstaaten : AT, BE, CH, DE, FR, GB, GR, IT, LI, NL, SE**

**1.** Dihydropyridinether der allgemeinen Formel (I)

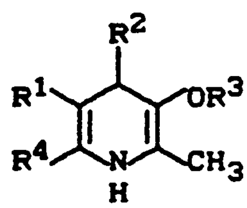

(I),

in welcher

$R^1$ - für Cyano, Nitro oder
- für eine Gruppe $-COOR^5$ steht,
worin

$R^5$ - geradkettiges, verzweigtes oder cyclisches Alkyl mit bis zu 6 Kohlenstoffatomen bedeutet, das durch ein Sauerstoffatom in der Kette unterbrochen sein kann, und das ein- oder mehrfach gleich oder verschieden durch Halogen, Cyano, Hydroxy, Phenyl, Phenoxy oder durch eine Gruppe der Formel

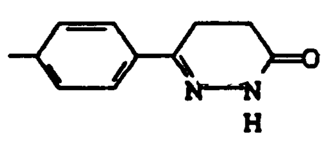

substituiert sein kann,

$R^2$ - für Aryl mit 6 bis 12 Kohlenstoffatomen steht, das bis zu 3-fach gleich oder verschieden durch Halogen, Cyano, Nitro, Trifluormethyl, Trifluormethoxy, Trifluormethylthio, Alkyl mit bis zu 4 Kohlenstoffatomen oder Alkoxy mit bis zu 4 Kohlenstoffatomen substituiert sein kann,

$R^3$ - für Aryl mit 6 bis 12 Kohlenstoffatomen steht, oder
- für geradkettiges, verzweigtes oder cyclisches Alkyl mit bis zu 6 Kohlenstoffatomen steht und

$R^4$ - für geradkettiges Alkyl mit bis 2 bis 4 Kohlenstoffatomen oder
- für Amino steht

und deren physiologisch unbedenkliche Salze.

**2.** Verbindungen der allgemeinen Formel (I) gemäß Anspruch 1, in welcher

$R^1$ - für Nitro oder
- für eine Gruppe $-COOR^5$ steht, worin

$R^5$ - geradkettiges oder verzweigtes Alkyl mit bis zu 4 Kohlenstoffatomen bedeutet, das durch ein Sauerstoffatom in der Kette unterbrochen sein kann, und das durch Fluor, Chlor, Brom, Cyano, Hydroxy, Phenyl oder durch eine Gruppe der Formel

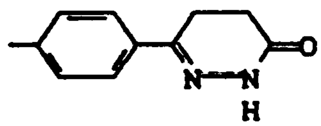

substituiert sein kann,

$R^2$ - für Phenyl steht, das bis zu 2-fach gleich oder verschieden durch Fluor, Chlor, Brom, Nitro, Trifluormethyl, Trifluormethoxy, Methyl oder Methoxy substituiert sein kann

$R^3$ - für Phenyl steht, oder
- für geradkettiges oder verzweigtes Alkyl mit bis zu 4 Kohlenstoffatomen,

und

$R^4$ - für geradkettiges Alkyl mit bis zu 4 Kohlenstoffatomen oder
- für Amino steht,

und deren physiologisch unbedenkliche Salze.

**3.** Verbindungen der allgemeinen Formel (I) gemäß Anspruch 1, in welcher

$R^1$ - für Nitro oder
- für eine Gruppe $-COOR^5$ steht

worin

$R^5$ - für geradkettiges oder verzweigtes Alkyl mit bis zu 3 Kohlenstoffatomen steht, das durch eine Gruppe der Formel

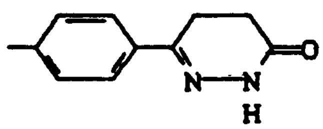

substituiert sein kann,

$R^2$ - für Phenyl steht, das bis zu 2-fach gleich oder verschieden durch Chlor, Nitro oder Trifluormethyl substituiert sein kann,

$R^3$ - für Phenyl oder Methyl,

und

$R^4$ - für Methyl oder
- für Amino steht

und deren physiologisch unbedenkliche Salze.

**4.** Verbindungen der allgemeinen Formel (I) gemäß Anspruch 1, zur Verwendung bei der Bekämpfung von Erkrankungen.

**5.** Verfahren zur Herstellung von Dihydropyridinethern der allgemeinen Formel (I)

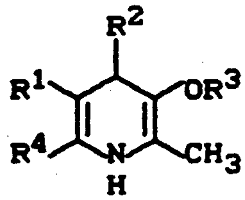

(I),

in welcher

$R^1$ - für Cyano, Nitro oder
- für eine Gruppe -$COOR^5$ steht,
worin

$R^5$ - geradkettiges, verzweigtes oder cyclisches Alkyl mit bis zu 6 Kohlenstoffatomen bedeutet, das durch ein Sauerstoffatom in der Kette unterbrochen sein kann, und das ein- oder mehrfach gleich oder verschieden durch Halogen, Cyano, Hydroxy, Phenyl, Phenoxy oder durch eine Gruppe der Formel

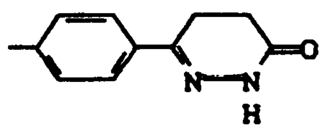

substituiert sein kann,

$R^2$ - für Aryl mit 6 bis 12 Kohlenstoffatomen steht, das bis zu 3-fach gleich oder verschieden durch Halogen, Cyano, Nitro, Trifluormethyl, Trifluormethoxy, Trifluormethylthio, Alkyl mit bis zu 4 Kohlenstoffatomen oder Alkoxy mit bis zu 4 Kohlenstoffatomen substituiert sein kann,

$R^3$ - für Aryl mit 6 bis 12 Kohlenstoffatomen steht, oder
- für geradkettiges, verzweigtes oder cyclisches Alkyl mit bis zu 6 Kohlenstoffatomen steht
und

$R^4$ - für geradkettiges Alkyl mit bis 2 bis 4 Kohlenstoffatomen oder
- für Amino steht

und deren physiologisch unbedenkliche Salze,
dadurch gekennzeichnet, daß man
[A] Aldehyde der allgemeinen Formel (II)

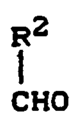

(II)

in welcher

$R^2$ - die oben angegebene Bedeutung hat

und Ketone der allgemeinen Formel (III)

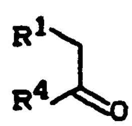

(III)

in welcher
$R^1$ und $R^4$ die oben angegebene Bedeutung haben,
oder deren Knoevenagel-Kondensationsprodukte (Ylidenverbindungen) der allgemeinen Formel (IV)

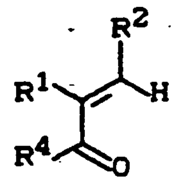

(IV)

in welcher
$R^1$, $R^2$ und $R^4$ die oben angegebene Bedeutung haben,
mit Enaminen der allgemeinen Formel (V)

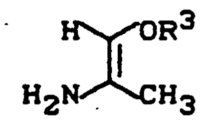

(V)

in welcher
$R^3$ die oben angegebene Bedeutung hat,
in inerten Lösemitteln umsetzt,
oder indem man
[B] Aldehyde der allgemeinen Formel (II) und Ketone der allgemeinen Formel (VI)

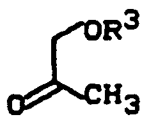

(VI)

in welcher
$R^3$ die oben angegebene Bedeutung hat,
oder deren Ylidenverbindungen der allgemeinen Formel (VII)

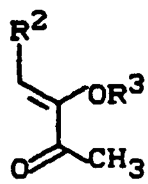

(VII)

in welcher
$R^2$ und $R^3$ die oben angegebene Bedeutung haben,
mit Ammoniak und Ketonen der allgemeinen Formel (III), oder mit Enaminen der allgemeinen Formel (VIII)

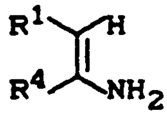

(VIII)

in welcher
$R^1$ und $R^4$ die oben angegebene Bedeutung haben,
in inerten Lösemitteln umsetzt.

6. Verfahren gemäß Anspruch 5, dadurch gekennzeichnet, daß man die Reaktion bei Temperaturen zwischen +20 ° C und 150 ° C in Gegenwart von inerten organischen Lösungsmitteln durchführt.

7. Arzneimittel, enthaltend mindestens eine Verbindung der allgemeinen Formel (I) gemäß Anspruch 1.

8. Verfahren zur Herstellung von Arzneimitteln, dadurch gekennzeichnet, daß man Verbindungen der allgemeinen Formel (I) gemäß Anspruch 1 gegebenenfalls unter Verwendung von üblichen Hilfs- und Trägerstoffen in eine geeignete Applikationsform überführt.

9. Verwendung von Verbindungen der allgemeinen Formel (I) gemäß Anspruch 1 bei der Herstellung von kreislaufwirksamen Arzneimitteln.

**Patentansprüche für folgenden Vertragsstaat : ES**

1. Verfahren zur Herstellung von Dihydropyridinethern der allgemeinen Formel (I)

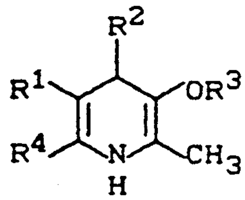

(I),

in welcher

$R^1$ - für Cyano, Nitro oder
- für eine Gruppe -$COOR^5$ steht,
worin

$R^5$ - geradkettiges, verzweigtes oder cyclisches Alkyl mit bis zu 6 Kohlenstoffatomen bedeutet, das durch ein Sauerstoffatom in der Kette unterbrochen sein kann, und das ein- oder mehrfach gleich oder verschieden durch Halogen, Cyano, Hydroxy, Phenyl, Phenoxy oder durch eine Gruppe der Formel

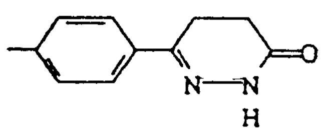

substituiert sein kann,

$R^2$ - für Aryl mit 6 bis 12 Kohlenstoffatomen steht, das bis zu 3-fach gleich oder verschieden durch Halogen, Cyano, Nitro, Trifluormethyl, Trifluormethoxy, Trifluormethylthio, Alkyl mit bis zu 4 Kohlenstoffatomen oder Alkoxy mit bis zu 4 Kohlenstoffatomen substituiert sein kann,

$R^3$ - für Aryl mit 6 bis 12 Kohlenstoffatomen steht, oder
- für geradkettiges, verzweigtes oder cyclisches Alkyl mit bis zu 6 Kohlenstoffatomen steht und

$R^4$ - für geradkettiges Alkyl mit bis 2 bis 4 Kohlenstoffatomen oder
- für Amino steht

und deren physiologisch unbedenkliche Salze,
dadurch gekennzeichnet, daß man
[A] Aldehyde der allgemeinen Formel (II)

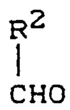

(II)

in welcher
$R^2$ - die oben angegebene Bedeutung hat
und Ketone der allgemeinen Formel (III)

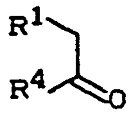

(III)

in welcher
$R^1$ und $R^4$ die oben angegebene Bedeutung haben,
oder deren Knoevenagel-Kondensationsprodukte (Ylidenverbindungen) der allgemeinen Formel (IV)

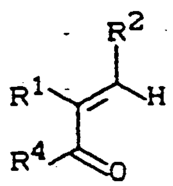

(IV)

in welcher
$R^1$, $R^2$ und $R^4$ die oben angegebene Bedeutung haben,
mit Enaminen der allgemeinen Formel (V)

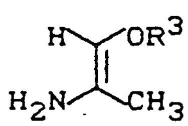

(V)

in welcher
$R^3$ die oben angegebene Bedeutung hat,
in inerten Lösemitteln umsetzt,
oder indem man
[B] Aldehyde der allgemeinen Formel (II) und Ketone der allgemeinen Formel (VI)

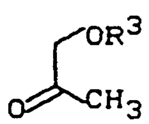

(VI)

in welcher
$R^3$ die oben angegebene Bedeutung hat,
oder deren Ylidenverbindungen der allgemeinen Formel (VII)

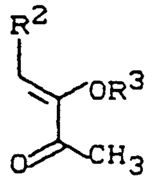

(VII)

in welcher
$R^2$ und $R^3$ die oben angegebene Bedeutung haben,
mit Ammoniak und Ketonen der allgemeinen Formel (III), oder mit Enaminen der allgemeinen Formel (VIII)

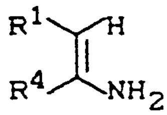

(VIII)

in welcher
$R^1$ und $R^4$ die oben angegebene Bedeutung haben,
in inerten Lösemitteln umsetzt.

**2.** Verfahren gemäß Anspruch 1, dadurch gekennzeichnet, daß man die Reaktion bei Temperaturen zwischen +20 °C und 150 °C in Gegenwart von inerten organischen Lösungsmitteln durchführt,

**3.** Verfahren zur Herstellung von Arzneimitteln, dadurch gekennzeichnet, daß man Verbindungen der allgemeinen Formel (I) gemäß Anspruch 1 gegebenenfalls unter Verwendung von üblichen Hilfs- und Trägerstoffen in eine geeignete Applikationsform überführt,

**Claims**

**Claims for the following Contracting States : AT, BE, CH, DE, FR, GB, GR, IT, LI, NL, SE**

**1.** Dihydropyridine ethers of the general formula (I)

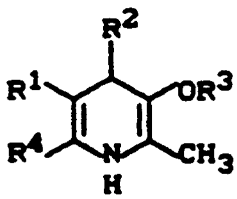

(I)

in which

$R^1$ - represents cyano, nitro or
- represents a -$COOR^5$ group,
wherein

$R^5$ - denotes straight-chain, branched or cyclic alkyl having up to 6 carbon atoms, which can be interrupted by an oxygen atom in the chain and which can be monosubstituted or polysubstituted by identical or different substituents from the series comprising halogen, cyano, hydroxyl, phenyl, phenoxy or by a group of the formula

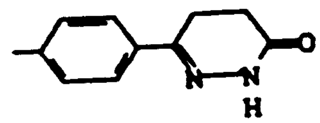

$R^2$ - represents aryl having 6 to 12 carbon atoms, which can be monosubstituted, disubstituted or trisubstituted by identical or different substituents from the series comprising halogen, cyano, nitro, trifluoromethyl, trifluoromethoxy, trifluoromethylthio, alkyl having up to 4 carbon atoms or alkoxy having up to 4 carbon atoms,

$R^3$ - represents aryl having 6 to 12 carbon atoms, or
- represents straight-chain, branched or cyclic alkyl having up to 6 carbon atoms
and

$R^4$ - represents straight-chain alkyl having 2 to 4 carbon atoms or
- represents amino

and their physiologically acceptable salts.

**2.** Compounds of the general formula (I) according to Claim 1, in which

$R^1$ - represents nitro or
- represents a -COOR$^5$ group,
wherein

$R^5$ - denotes straight-chain or branched alkyl having up to 4 carbon atoms, which can be interrupted by an oxygen atom in the chain and which can be substituted by fluorine, chlorine, bromine, cyano, hydroxyl, phenyl or by a group of the formula

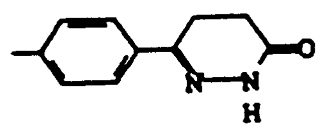

$R^2$ - represents phenyl which can be monosubstituted or disubstituted by identical or different substituents from the series comprising fluorine, chlorine, bromine, nitro, trifluoromethyl, trifluoromethoxy, methyl or methoxy,

$R^3$ - represents phenyl, or
- represents straight-chain or branched alkyl having up to 4 carbon atoms,
and

$R^4$ - represents straight-chain alkyl having up to 4 carbon atoms or
- represents amino

and their physiologically acceptable salts.

**3.** Compounds of the general formula (I) according to Claim 1, in which

$R^1$ - represents nitro or
- represents a -COOR$^5$ group,
wherein

$R^5$ - represents straight-chain or branched alkyl having up to 3 carbon atoms, which can be substituted by a group of the formula

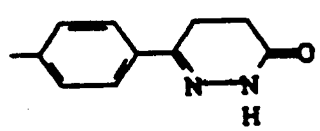

$R^2$ - represents phenyl which can be monosubstituted or disubstituted by identical or different substituents from the series comprising chlorine, nitro or trifluoromethyl,

$R^3$ - represents phenyl or methyl,
and

$R^4$ - represents methyl or
- represents amino
and their physiologically acceptable salts.

**4.** Compounds of the general formula (I) according to Claim 1 for use in controlling disorders.

**5.** Process for the preparation of dihydropyridine ethers of the general formula (I)

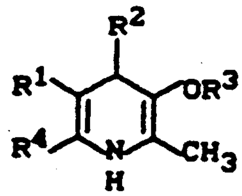

(I)

in which
$R^1$ - represents cyano, nitro or
- represents a -$COOR^5$ group,
wherein
$R^5$ - denotes straight-chain, branched or cyclic alkyl having up to 6 carbon atoms, which can be interrupted by an oxygen atom in the chain and which can be monosubstituted or polysubstituted by identical or different substituents from the series comprising halogen, cyano, hydroxyl, phenyl, phenoxy or by a group of the formula

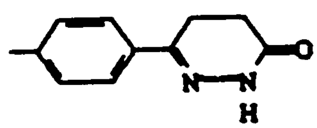

$R^2$ - represents aryl having 6 to 12 carbon atoms, which can be monosubstituted, disubstituted or trisubstituted by identical or different substituents from the series comprising halogen, cyano, nitro, trifluoromethyl, trifluoromethoxy, trifluoromethylthio, alkyl having up to 4 carbon atoms or alkoxy having up to 4 carbon atoms,
$R^3$ - represents aryl having 6 to 12 carbon atoms, or
- represents straight-chain, branched or cyclic alkyl having up to 6 carbon atoms
and
$R^4$ - represents straight-chain alkyl having 2 to 4 carbon atoms or
- represents amino
and their physiologically acceptable salts,
characterised in that
[A] aldehydes of the general formula (II)

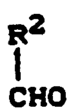

(II)

in which
$R^2$ - has the abovementioned meaning
and ketones of the general formula (III)

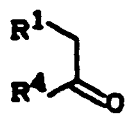

(III)

in which
$R^1$ and $R^4$ have the abovementioned meaning, or their Knoevenagel condensation products (ylidene compounds) of the general formula (IV)

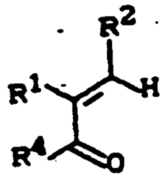

(IV)

in which
$R^1$, $R^2$ and $R^4$ have the abovementioned meaning,
are reacted with enamines of the general formula (V)

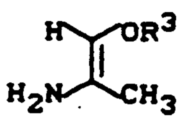

(V)

in which
$R^3$ has the abovementioned meaning
in inert solvents,
or
[B] aldehydes of the general formula (II) and ketones of the general formula (VI)

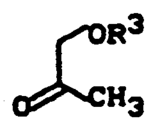

(VI)

in which
$R^3$ has the abovementioned meaning,
or their ylidene compounds of the general formula (VII)

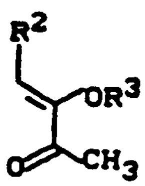

(VII)

in which
$R^2$ and $R^3$ have the abovementioned meaning,
are reacted with ammonia and ketones of the general formula (III), or with enamines of the general formula (VIII)

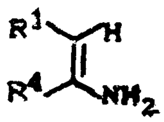

(VIII)

in which
$R^1$ and $R^4$ have the abovementioned meaning,
in inert solvents.

6. Process according to Claim 5, characterised in that the reaction is carried out at temperatures between +20 ° C and 150 ° C in the presence of inert organic solvents.

7. Medicaments containing at least one compound of the general formula (I) according to Claim 1.

8. Method for the production of medicaments, characterised in that compounds of the general formula (I) according to Claim 1 are converted into a suitable form for administration if appropriate using customary auxiliaries and excipients.

9. Use of compounds of the general formula (I) according to Claim 1 in the production of circulatory-active medicaments.

**Claims for the following Contracting State : ES**

1. Process for the preparation of dihydropyridine ethers of the general formula (I)

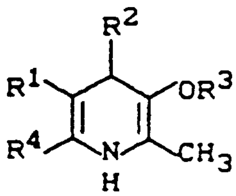

(I)

in which

$R^1$ - represents cyano, nitro or
- represents a -COOR$^5$ group,
wherein

$R^5$ - denotes straight-chain, branched or cyclic alkyl having up to 6 carbon atoms, which can be interrupted by an oxygen atom in the chain and which can be monosubstituted or polysubstituted by identical or different substituents from the series comprising halogen, cyano, hydroxyl, phenyl, phenoxy or by a group of the formula

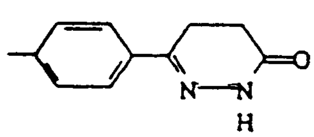

$R^2$ - represents aryl having 6 to 12 carbon atoms, which can be monosubstituted, disubstituted or trisubstituted by identical or different substituents from the series comprising halogen, cyano, nitro, trifluoromethyl, trifluoromethoxy, trifluoromethylthio, alkyl having up to 4 carbon atoms or alkoxy having up to 4 carbon atoms,

$R^3$ - represents aryl having 6 to 12 carbon atoms, or
- represents straight-chain, branched or cyclic alkyl having up to 6 carbon atoms
and

$R^4$ - represents straight-chain alkyl having 2 to 4 carbon atoms or
- represents amino

and their physiologically acceptable salts,
characterised in that
[A] aldehydes of the general formula (II)

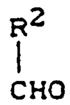

(II)

in which
$R^2$ - has the abovementioned meaning
and ketones of the general formula (III)

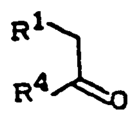

(III)

in which
$R^1$ and $R^4$ have the abovementioned meaning, or their Knoevenagel condensation products (ylidene compounds) of the general formula (IV)

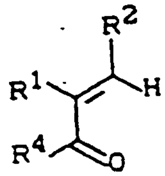

(IV)

in which
$R^1$, $R^2$ and $R^4$ have the abovementioned meaning,
are reacted with enamines of the general formula (V)

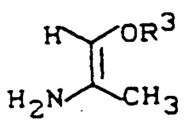

(V)

in which
$R^3$ has the abovementioned meaning
in inert solvents,
or
[B] aldehydes of the general formula (II) and ketones of the general formula (VI)

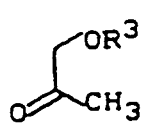

(VI)

in which
$R^3$ has the abovementioned meaning,
or their ylidene compounds of the general formula (VII)

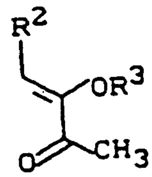

(VII)

in which
$R^2$ and $R^3$ have the abovementioned meaning,
are reacted with ammonia and ketones of the general formula (III), or with enamines of the general formula (VIII)

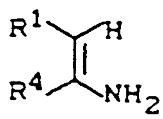

(VIII)

in which
$R^1$ and $R^4$ have the abovementioned meaning,
in inert solvents.

**2.** Process according to Claim 1, characterised in that the reaction is carried out at temperatures between +20 ° C and 150 ° C in the presence of inert organic solvents.

**3.** Method for the production of medicaments, characterised in that compounds of the general formula (I) according to Claim 1 are converted into a suitable form for administration if appropriate using customary auxiliaries and excipients.

**Revendications**

**Revendications pour les Etats contractants suivants : AT, BE, CH, DE, FR, GB, GR, IT, LI, NL, SE**

**1.** Ethers dihydropyridiniques de formule générale (I)

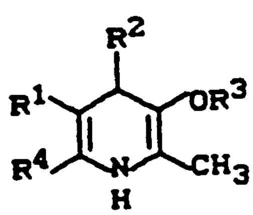

(I),

dans laquelle
$R^1$ - représente un groupe cyano, nitro, ou
- un groupe -$COOR^5$,
dans lequel
$R^5$ - signifie un groupe alkyle à chaîne linéaire ou ramifiée ou cyclique avec jusqu'à 6 atomes de carbone, pouvant être interrompu par un atome d'oxygène dans la chaîne et pouvant être substitué une ou plusieurs fois de manière identique ou différente par un halogène, un groupe cyano, hydroxy, phényl, phénoxy, ou par un groupe de formule

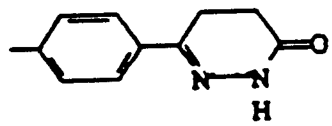

$R^2$ - représente un groupe aryle avec 6 à 12 atomes de carbone, qui peut être substitué jusqu'à 3 fois de manière identique ou différente par un halogène, un groupe cyano, nitro, trifluorométhyle, trifluorométhoxy, trifluorométhylthio, alkyle avec jusqu'à 4 atomes de carbone ou alcoxy avec jusqu'à 4 atomes de carbone,

$R^3$ - représente un groupe aryle avec 6 à 12 atomes de carbone, ou
- un groupe alkyle à chaîne linéaire ou ramifiée ou cyclique avec jusqu'à 6 atomes de carbone,

et

$R^4$ - représente un groupe alkyle à chaîne linéaire avec 2 à 4 atomes de carbone, ou
- un groupe amino

et leurs sels non dangereux du point de vue physiologique.

**2.** Composés de formule générale (I) selon la revendication 1, dans lesquels

$R^1$ - représente un groupe nitro ou
- un groupe $COOR^5$

dans lequel

$R^5$ - représente un groupe alkyle à chaîne droite ou ramifiée avec jusqu'à 4 atomes de carbone, qui peut être interrompu par un atome d'oxygène dans la chaîne, et qui peut être substitué par un atome de fluor, de chlore, de brome, un groupe cyano, hydroxy, phényle, ou par un groupe de formule

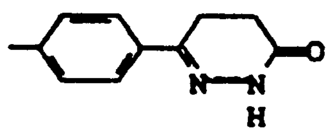

$R^2$ - représente un groupe phényle qui peut être substitué jusqu'à 2 fois de manière identique ou différente par un atome de fluor, de chlore, de brome, un groupe nitro, trifluorométhyle, trifluorométhoxy, méthyle ou méthoxy

$R^3$ - signifie un groupe phényle, ou
- un groupe alkyle à chaîne linéaire ou ramifiée avec jusqu'à 4 atomes de carbone,

et

$R^4$ - représente un groupe alkyle à chaîne linéaire avec jusqu'à 4 atomes de carbone ou
- un groupe amino,

et leurs sels inoffensifs du point de vue physiologique.

**3.** Composés de formule générale (I) selon la revendication 1, dans lesquels

$R^1$ - représente un groupe nitro ou
- un groupe $-COOR^5$

dans lequel

$R^5$ - représente un groupe alkyle à chaîne linéaire ou ramifiée avec jusqu'à 3 atomes de carbone, qui peut être substitué par un groupe de formule

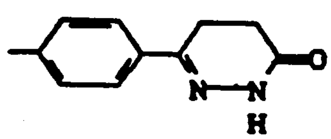

$R^2$ - représente un groupe phényle qui peut être substitué jusqu'à 2 fois de manière identique ou

différente par un atome de chlore, un groupe nitro ou trifluorométhyle,

$R^3$ - représente un groupe phényle ou méthyle,

et

$R^4$ - représente un groupe méthyle ou

- un groupe amino

et leurs sels physiologiquement inoffensifs.

**4.** Composés de formule générale (I) selon la revendication 1, pour l'application dans la lutte contre les maladies.

**5.** Procédé pour fabriquer des éthers dihydropyridiniques de formule générale (I)

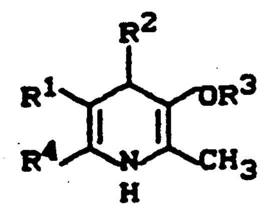

(I),

dans laquelle

$R^1$ - représente un groupe cyano, nitro, ou

- un groupe $-COOR^5$,

dans lequel

$R^5$ - signifie un groupe alkyle à chaîne linéaire ou ramifiée ou cyclique avec jusqu'à 6 atomes de carbone, pouvant être interrompu par un atome d'oxygène dans la chaîne et pouvant être substitué une ou plusieurs fois de manière identique ou différente par un halogène, un groupe cyano, hydroxy, phényl, phénoxy, ou par un groupe de formule

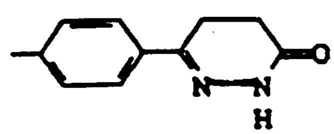

$R^2$ - représente un groupe aryle avec 6 à 12 atomes de carbone, qui peut être substitué jusqu'à 3 fois de manière identique ou différente par un halogène, un groupe cyano, nitro, trifluorométhyle, trifluorométhoxy, trifluorométhylthio, alkyle avec jusqu'à 4 atomes de carbone ou alcoxy avec jusqu'à 4 atomes de carbone,

$R^3$ - représente un groupe aryle avec 6 à 12 atomes de carbone, ou

- un groupe alkyle à chaîne linéaire ou ramifiée ou cyclique avec jusqu'à 6 atomes de carbone,

et

$R^4$ - représente un groupe alkyle à chaîne linéaire avec 2 à 4 atomes de carbone, ou

- un groupe amino

et leurs sels non dangereux du point de vue physiologique,

caractérisé en ce que

[A] l'on fait réagir dans des solvants inertes des aldéhydes de formule générale (II)

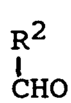

(II)

dans laquelle

$R^2$ - possède la signification décrite ci-dessus

et des cétones de formule générale (III)

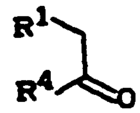

(III)

dans laquelle
$R^1$ et $R^4$ possèdent les significations décrites ci-dessus,
ou leurs produits de condensation de Knoevenagel (composés ylidéniques) de formule générale (IV)

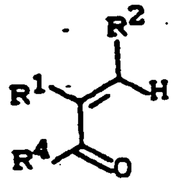

(IV)

dans laquelle
$R^1$, $R^2$ et $R^4$ possèdent les significations décrites ci-dessus,
avec des énamines de formule générale (V)

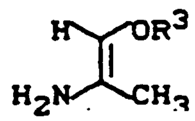

(V)

dans laquelle
$R^3$ possède la signification décrite ci-dessus,
ou
[B] l'on fait réagir dans des solvants inertes des aldéhydes de formule générale (II) et des cétones de formule générale (VI)

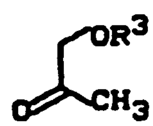

(VI)

dans laquelle
$R^3$ possède la signification décrite ci-dessus,
ou leurs composés ylidéniques de formule générale (VII)

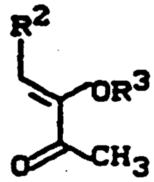

(VII)

dans laquelle
$R^2$ et $R^3$ possèdent les significations décrites ci-dessus,
avec de l'ammoniaque et des cétones de formule générale (III) ou avec des énamines de formule générale (VIII)

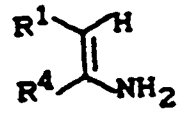

(VIII)

dans laquelle
$R^1$ et $R^4$ possèdent les significations décrites ci-dessus.

6. Procédé selon la revendication 1, caractérisé en ce qu'on effectue la réaction à des températures de +20 ° C à 150 ° C en présence de solvants organiques inertes.

7. Médicaments contenant au moins un composé de formule générale (I) selon la revendication 1.

8. Procédé pour la fabrication de médicaments, caractérisé en ce que l'on met des composés de formule générale (I) selon la revendication 1 dans une forme d'application appropriée, éventuellement en utilisant des adjuvants et des substances de support.

9. Utilisation des composés de formule générale (I) selon la revendication 1 dans la fabrication de médicaments agissant sur la circulation.

**Revendications pour l'Etat contractant suivant : ES**

1. Procédé pour fabriquer des éthers dihydropyridiniques de formule générale (I)

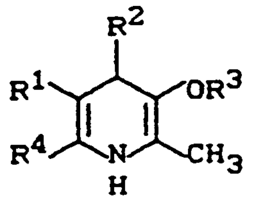

(I),

dans laquelle

$R^1$ - représente un groupe cyano, nitro, ou
- un groupe -COOR$^5$,
dans lequel

$R^5$ - signifie un groupe alkyle à chaîne linéaire ou ramifiée ou cyclique avec jusqu'à 6 atomes de carbone, pouvant être interrompu par un atome d'oxygène dans la chaîne et pouvant être substitué une ou plusieurs fois de manière identique ou différente par un halogène, un groupe cyano, hydroxy, phényl, phénoxy, ou par un groupe de formule

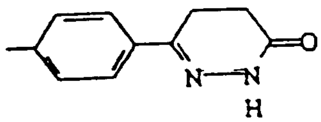

$R^2$ - représente un groupe aryle avec 6 à 12 atomes de carbone, qui peut être substitué jusqu'à 3 fois de manière identique ou différente par un halogène, un groupe cyano, nitro, trifluorométhyle, trifluorométhoxy, trifluorométhylthio, alkyle avec jusqu'à 4 atomes de carbone ou alcoxy avec jusqu'à 4 atomes de carbone,

$R^3$ - représente un groupe aryle avec 6 à 12 atomes de carbone, ou
- un groupe alkyle à chaîne linéaire ou ramifiée ou cyclique avec jusqu'à 6 atomes de carbone,
et

$R^4$ - représente un groupe alkyle à chaîne linéaire avec 2 à 4 atomes de carbone, ou

- un groupe amino

et leurs sels non dangereux du point de vue physiologique,
caractérisé en ce que
[A] l'on fait réagir dans des solvants inertes des aldéhydes de formule générale (II)

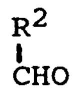

(II)

dans laquelle

$R^2$ - possède la signification décrite ci-dessus

et des cétones de formule générale (III)

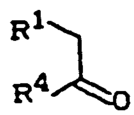

(III)

dans laquelle
$R^1$ et $R^4$ possèdent les significations décrites ci-dessus,
ou leurs produits de condensation de Knoevenagel (composés ylidéniques) de formule générale (IV)

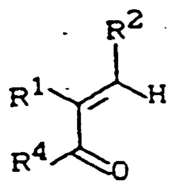

(IV)

dans laquelle
$R^1$, $R^2$ et $R^4$ possèdent les significations décrites ci-dessus,
avec des énamines de formule générale (V)

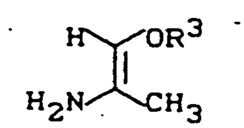

(V)

dans laquelle
$R^3$ possède la signification décrite ci-dessus,
ou
[B] l'on fait réagir dans des solvants inertes des aldéhydes de formule générale (II) et des cétones de formule générale (VI)

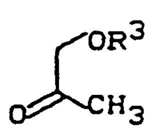

(VI)

dans laquelle
$R^3$ possède la signification décrite ci-dessus,

ou leurs composés ylidéniques de formule générale (VII)

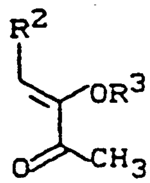

(VII)

dans laquelle
$R^2$ et $R^3$ possèdent les significations décrites ci-dessus,
avec de l'ammoniaque et des cétones de formule générale (III) ou avec des énamines de formule générale (VIII)

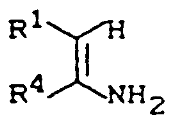

(VIII)

dans laquelle
$R^1$ et $R^4$ possèdent les significations décrites ci-dessus.

**2.** Procédé selon la revendication 1, caractérisé en ce qu'on effectue la réaction à des températures de +20 ° C à 150 ° C en présence de solvants organiques inertes.

**3.** Procédé pour la fabrication de médicaments, caractérisé en ce que l'on met des composés de formule générale (I) selon la revendication 1 dans une forme d'application appropriée, éventuellement en utilisant des adjuvants et des substances de support.